# FASCICULE DE BREVET EUROPEEN

(11) **EP 3 921 005 B1**
(45) Date de publication et mention de la délivrance du brevet: **14.06.2023**
(21) Numéro de dépôt: 20702032.2
(22) Date de dépôt: 31.01.2020
(51) Int. Cl.: A61M 16/00, A61M 16/08

(54) **DISPOSITIF DE VENTILATION RESPIRATOIRE**
BEATMUNGSVORRICHTUNG
RESPIRATORY VENTILATION DEVICE

(30) Priorité: 04.02.2019 FR 1901084
(43) Date de publication de la demande: 15.12.2021
(73) Titulaire: Sleepinnov Technology, 38430 Moirans (FR)
(72) Inventeur: HUNGR, Nikolaï, 38430 Moirans (FR); ARGOD, Jérôme, 38430 Moirans (FR)
(74) Mandataire: INNOV-GROUP
(86) Numéro de dépôt international: PCT/EP2020/052457
(87) Numéro de publication internationale: WO 2020/161019

(56) Documents cités:
- EP-A1- 2 822 626
- EP-A1- 2 822 626
- EP-A1- 2 923 721
- US-B1- 7 975 688
- US-B2- 8 453 640

## Description

### DOMAINE TECHNIQUE

Le domaine technique de l'invention est un dispositif de ventilation par pression positive continue. Ce type de dispositif est couramment utilisé dans le traitement de l'apnée du sommeil.

### ART ANTERIEUR

Le recours à la ventilation par pression positive continue (PPC) constitue un traitement de référence dans le domaine de l'apnée du sommeil. Ce traitement consiste à insuffler de l'air de façon continue dans un masque appliqué sur le visage d'un utilisateur. Il peut s'agir d'un masque nasal, narinaire ou facial. Le souffle d'air parvient aux voies respiratoires de l'utilisateur, en exerçant une pression suffisante sur ces dernières, de façon à prévenir la formation d'un collapsus.

Les dispositifs de ventilation positive par PPC sont utilisés la nuit. Par conséquent, ils doivent être le plus silencieux possible, sachant qu'il existe deux sources de bruit :
- la propagation de vibrations mécaniques générées par le ventilateur : il s'agit de vibrations susceptibles de se propager à travers les parties solides du dispositif, en particulier les parois.
- l'écoulement de l'air à travers le dispositif : en amont du ventilateur, l'air est aspiré tandis qu'en aval du ventilateur, l'air est soufflé.

Ainsi, le bruit a une origine soit mécanique, soit aéraulique.

Par ailleurs, le dispositif peut être destiné à être transporté et il est important qu'il soit suffisamment compact pour être disposé dans un bagage. Il doit également être simple d'utilisation. En outre, le coût de fabrication doit être réduit : la conception du dispositif doit donc être aussi simple que possible.

Parmi les dispositifs de l'art antérieur, citons par exemple le dispositif décrit dans EP2923721. Ce dernier est destiné à être disposé sur un support horizontal. Mais l'emprise horizontale de ce dispositif est relativement importante, en particulier lorsque le dispositif comporte un module d'humidification. Un dispositif plus compact a été décrit dans EP2822626. Il comporte une chambre de ventilation maintenue, à l'intérieur d'une enceinte, par une membrane en élastomère. La membrane en élastomère s'étend entre une périphérie, fixée à l'enceinte du dispositif, et la chambre de ventilation. La chambre de ventilation est insérée dans une ouverture ménagée au centre de la membrane. Dans la partie centrale, la membrane se dédouble en une partie inférieure et une partie supérieure, de façon à former une enveloppe s'étendant de part et d'autre de la chambre de ventilation. Au niveau de la périphérie de la membrane, cette dernière comporte un soufflet, de façon à atténuer une transmission de vibrations, générées par la chambre de ventilation, vers l'enceinte rigide. Afin de prévenir un contact entre la chambre de ventilation et l'enceinte, des cônes sont ménagés, permettant d'absorber d'éventuels chocs lors de mouvements axiaux de la chambre de ventilation. Le dédoublement de la membrane, la présence d'un soufflet et la disposition de cônes constituent des éléments complexifiant la réalisation de la membrane, et accentuant la fragilité de cette dernière. Le document US7975688 décrit également une chambre de ventilation maintenue par une membrane en élastomère.

Parmi les dispositifs de l'art antérieur, citons également US8453640, décrivant un dispositif de respiration compact.

L'inventeur a conçu un dispositif de respiration ventilatoire, en portant une attention particulière sur la réduction du bruit durant le fonctionnement, qu'il s'agisse des vibrations mécaniques induites par le fonctionnement du ventilateur ou du bruit de l'écoulement d'air. En outre, le dispositif objet de l'invention est compact, robuste et simple à fabriquer. Sa conception le rend aisément transportable.

Le document US7975688B1 divulgue un dispositif de respiration ventilatoire.

### EXPOSE DE L'INVENTION

Un objet de l'invention est un dispositif de ventilation respiratoire, destiné à envoyer un flux d'air, généré par un ventilateur, dans un conduit, le conduit s'étendant entre le dispositif et un masque respiratoire, destiné à être utilisé par un utilisateur, le dispositif comportant une enceinte, s'étendant autour d'un axe longitudinal et comprenant :
- une entrée d'air, destinée à admettre l'air dans l'enceinte ;
- une zone amont de circulation d'air s'étendant entre l'entrée d'air et le ventilateur ;
- une sortie d'air, la sortie d'air étant configurée pour être raccordée au conduit ;
de telle sorte que lorsque le ventilateur fonctionne, l'air s'écoule depuis l'entrée d'air successivement à travers la zone amont de circulation d'air, le ventilateur et la sortie d'air, le dispositif étant tel que :
- le ventilateur est disposé dans une chambre de ventilation;
- la chambre de ventilation est maintenue par une première membrane souple, s'étendant entre une première périphérie et la chambre de ventilation, la première périphérie étant maintenue fixe par rapport à l'enceinte, la première membrane définissant une première ouverture centrale, à travers laquelle s'étend la chambre de ventilation, ou un support relié à la chambre de ventilation.

Le dispositif peut comporter une des caractéristiques suivantes, prises isolément ou selon les combinaisons techniquement réalisables.
- la chambre de ventilation est maintenue par une deuxième membrane souple, distincte de la première membrane, la deuxième membrane s'étendant entre une deuxième périphérie et la chambre de ventilation, la deuxième périphérie étant maintenue fixe par rapport à l'enceinte, à une distance non nulle de la première périphérie, la deuxième membrane comportant une deuxième ouverture centrale, à travers laquelle s'étend la chambre de ventilation ou un support relié à la chambre de ventilation.
- La chambre de ventilation comporte une portion d'admission, un carter du ventilateur, une portion d'évacuation et un compartiment moteur ;
- La première membrane s'étend entre la première périphérie et le carter du ventilateur ou entre la première périphérie et le compartiment moteur ;
- La deuxième membrane s'étend entre la deuxième périphérie et la portion d'admission;
- Le dispositif comporte une troisième membrane, s'étendant entre une troisième périphérie, fixe par rapport à l'enceinte, et/ou fixe par rapport à la coque, et la portion d'évacuation.
- Le dispositif comporte une coque, de préférence rigide, s'étendant autour d'un axe central, la coque étant disposée dans l'enceinte en étant avantageusement fixe par rapport à cette dernière, la coque s'étendant autour de la chambre de ventilation, la coque comportant une ouverture d'admission, permettant une admission de l'air dans la coque, et une ouverture d'extraction, permettant l'extraction de l'air de la coque. Ainsi, la zone amont de circulation d'air s'étend :
   ▪ à l'extérieur de la coque, entre l'entrée d'air de l'enceinte et l'ouverture d'admission;
   ▪ à l'intérieur de la coque, entre l'ouverture d'admission et la chambre de ventilation.
- La coque comporte un fond de coque, en face de l'ouverture d'admission. Le fond de coque peut faire face à la portion d'admission de la chambre de ventilation, le fond de coque formant un déflecteur d'air entre l'ouverture d'admission et la portion d'admission.
- L'axe central de la coque correspond à l'axe longitudinal ; dans ce cas, l'ouverture d'admission et le fond de coque sont de préférence alignés selon l'axe longitudinal.
- La première membrane est maintenue par la coque, et s'étend entre la coque et la chambre de ventilation, la première membrane s'étendant, au moins en partie, perpendiculairement à l'axe central de la coque.
- La première membrane forme une première cloison à l'intérieur de la coque, la première membrane comportant une ouverture de façon à permettre un passage de l'air de part et d'autre de la cloison.
- La deuxième membrane est maintenue par la coque, et s'étend entre la coque et la chambre de ventilation, la deuxième membrane s'étendant, au moins en partie, perpendiculairement à l'axe central de la coque.
- La deuxième membrane forme une deuxième cloison à l'intérieur de la coque, la deuxième membrane comportant une ouverture de façon à permettre un déplacement de l'air de part et d'autre de la cloison.
- La portion d'évacuation de la chambre de ventilation :
   ▪ débouche dans l'ouverture d'extraction de la coque ;
   ▪ ou s'étend à travers l'ouverture d'extraction de la coque.
- Le dispositif comporte une chambre d'expansion amont, adjacente de l'ouverture d'admission de la coque, la chambre d'expansion amont comportant une entrée, une partie centrale, et une sortie, agencées autour d'un axe amont, la section de la partie centrale, perpendiculairement à l'axe amont, étant supérieure aux sections respectives de l'entrée et de la sortie de la chambre d'expansion amont. La chambre d'expansion amont peut déboucher sur l'ouverture d'admission de la coque.
- Le dispositif comporte une chambre d'expansion aval, adjacente de la portion d'admission de la chambre de ventilation, la chambre d'expansion aval comportant une entrée, une partie centrale, et une sortie, agencées autour d'un axe aval, la section de la partie centrale, perpendiculairement à l'axe aval, étant supérieure aux sections respectives de l'entrée et de la sortie de la chambre d'expansion aval.
- Le dispositif est tel que la chambre d'expansion aval débouche sur la portion d'admission de la chambre de ventilation.
- Le dispositif est tel que la deuxième membrane s'étend entre la deuxième périphérie et la chambre d'expansion aval, de telle sorte que la portion d'admission est maintenue par la deuxième membrane.
- La première membrane et la deuxième membrane sont souples et formées d'un matériau élastomère. Il en est de même de l'éventuelle troisième membrane.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

### FIGURES

La figure 1A est un schéma montrant une utilisation du dispositif.
Les figures 1B et 1C sont des vues de l'enceinte du dispositif.
La figure 1D montre une partie de l'enceinte du dispositif, à laquelle on a retiré un volet amovible.
La figure 1E montre des composants du dispositif situés à l'intérieur de l'enceinte.
Les figures 2A et 2B représentent une coque située dans l'enceinte, ainsi qu'un tube d'arrivée et un tube de sortie d'air.
La figure 2C montre un exemple de chambre d'expansion amont.
La figure 2D représente la chambre de ventilation.
La figure 2E montre la première membrane, la deuxième membrane ainsi que la troisième membrane, ces membranes permettant un maintien de la chambre de ventilation à l'intérieur de la coque.
La figure 2F montre la chambre de ventilation ainsi qu'une chambre d'expansion aval.
La figure 2G montre la première membrane, la deuxième membrane ainsi que la troisième membrane, ces membranes permettant un maintien de la chambre de ventilation à l'intérieur de la coque.
La figure 3A montre la première membrane.
La figure 3B montre la deuxième membrane.
La figure 4A montre les principaux composants de l'enceinte, à travers lesquels s'écoule l'air. Il en est de même de la figure 4B.
La figure 5 est un autre mode de réalisation du dispositif.

### EXPOSE DE MODES DE REALISATION PARTICULIERS

Dans la description qui suit, les termes amont/aval sont à interpréter selon le sens de l'écoulement de l'air.

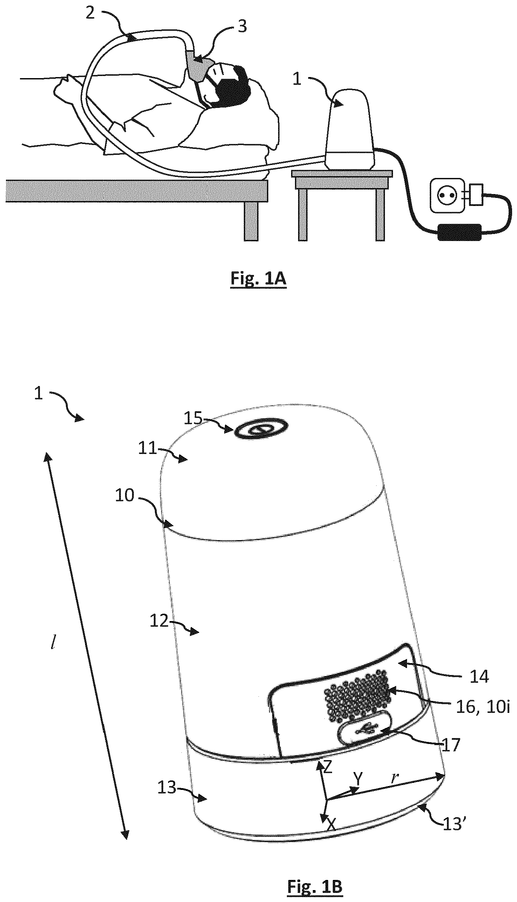

La figure 1A montre un dispositif 1 d'aide à la respiration selon l'invention. Le dispositif comporte un conduit 2, le reliant à un masque respiratoire 3 destiné à être appliqué sur le visage d'un utilisateur. De préférence, le conduit 2 est un conduit souple, dont la longueur est de quelques mètres. Le dispositif 1 comporte un ventilateur, commandé pour maintenir une pression de consigne au niveau du masque respiratoire. Il est essentiellement destiné à un usage nocturne. Lors de son utilisation, le dispositif peut notamment être disposé sur un support plan, par exemple une table de chevet. En cela, son emprise horizontale doit être minimisée.

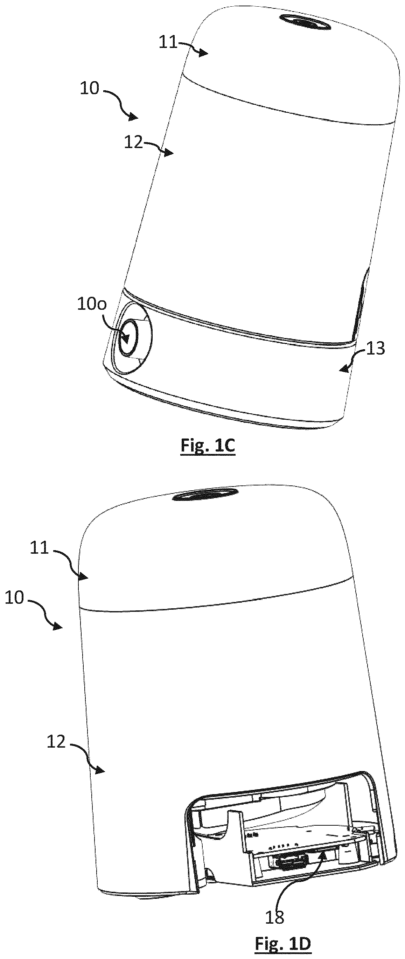

Les figures 1B et 1C montrent une enceinte 10 du dispositif 1. L'enceinte 10 s'étend selon un axe longitudinal Z, en définissant une longueur *l*. Elle s'étend radialement selon un plan de base XY, dans lequel elle définit un rayon *r.* La longueur *l* est typiquement comprise entre 10 cm et 40cm, tandis que le rayon r est généralement compris entre 5 cm et 30 cm. Les dimensions du dispositif le rendent aisément transportable. Par ailleurs, le dispositif est adapté à être déposé sur un support plan de faibles dimensions, par exemple une table. Lors de l'utilisation, l'axe longitudinal Z est de préférence parallèle à la verticale, tandis que le plan de base XY est horizontal.

L'enceinte 10 est de préférence constituée d'un matériau rigide, par exemple un plastique. Dans l'exemple représenté, l'enceinte 10 comporte un tronçon supérieur 11, un tronçon intermédiaire 12 et un tronçon inférieur 13. Le tronçon inférieur est délimité par une surface inférieure 13', de préférence plane et parallèle au plan de base XY. Le tronçon intermédiaire 12 comporte une grille 16, agissant en tant que filtre, et formant une entrée d'air 10i, à travers laquelle l'air peut pénétrer à l'intérieur de l'enceinte 10. Le tronçon intermédiaire 12 comporte également une embase de connexion 17, permettant un raccordement du dispositif 1 à une unité de traitement distante, de façon à paramétrer une unité de contrôle 18 disposée à l'intérieur de l'enceinte 10 et décrit en lien avec la figure 1D.

Dans cet exemple, l'embase de connexion 17 et l'entrée d'air 10i sont disposées sur un volet amovible 14.

Sur le tronçon supérieur 11 est disposé un commutateur de commande marche/arrêt 15. La disposition au sommet de l'enceinte 10 le rend aisément accessible par un utilisateur, y compris dans la pénombre ou l'obscurité. Le commutateur de commande 15 peut comporter une source de lumière, par exemple une diode électroluminescente, de façon à être visible dans l'obscurité. L'enceinte 10 est de préférence symétrique autour de l'axe longitudinal Z. Le commutateur de commande marche/arrêt est alors centré autour de l'axe longitudinal Z. Ainsi, quelle que soit la rotation du dispositif 1, autour de l'axe longitudinal Z, le commutateur marche/arrêt 15 ne bouge pas : sa position est indépendante de la rotation du dispositif Z autour de l'axe longitudinal Z.

Sur la figure 1C, on observe que le tronçon inférieur 13 comporte une ouverture de sortie 10o, formant une sortie d'air du dispositif 1. L'ouverture de sortie 10o est destinée à être raccordée au conduit 2, représenté sur la figure 1A, lors de l'utilisation du dispositif 1.

Selon une variante, le tronçon inférieur 13 comporte une capacité d'eau, formant un humidificateur. L'air insufflé par le dispositif 1 s'écoule, dans le tronçon inférieur 13, en dessus de la capacité d'eau de façon à s'humidifier, en amont de l'ouverture 10o. La disposition de l'humidificateur à l'intérieur du tronçon inférieur permet de ne pas modifier l'encombrement dans le plan XY.

La figure 1D montre le tronçon intermédiaire 12 et le tronçon supérieur 11. Sur la figure 1C, le volet 14 a été retiré. On observe une unité de contrôle 18, permettant le paramétrage et/ou le pilotage du dispositif 1, et en particulier la commande du ventilateur. L'unité de contrôle 18 peut être paramétrée par une liaison filaire raccordée à l'embase 17, comme précédemment décrit.

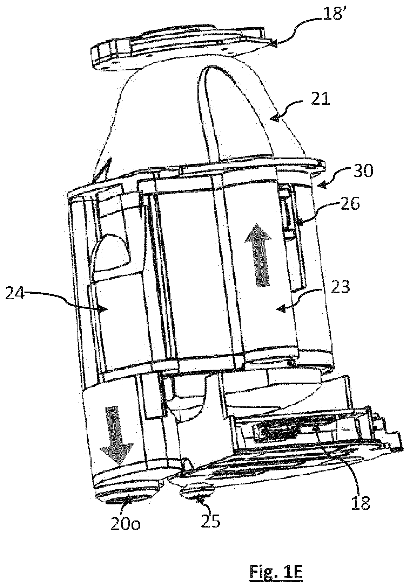

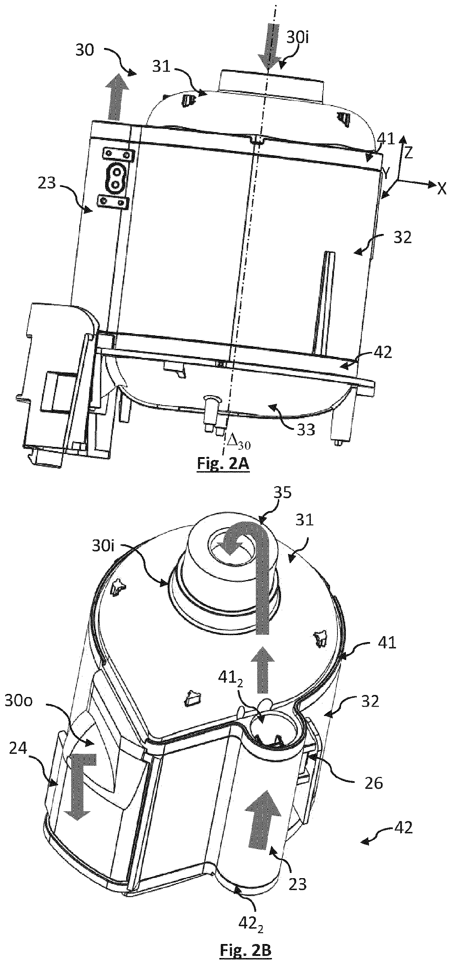

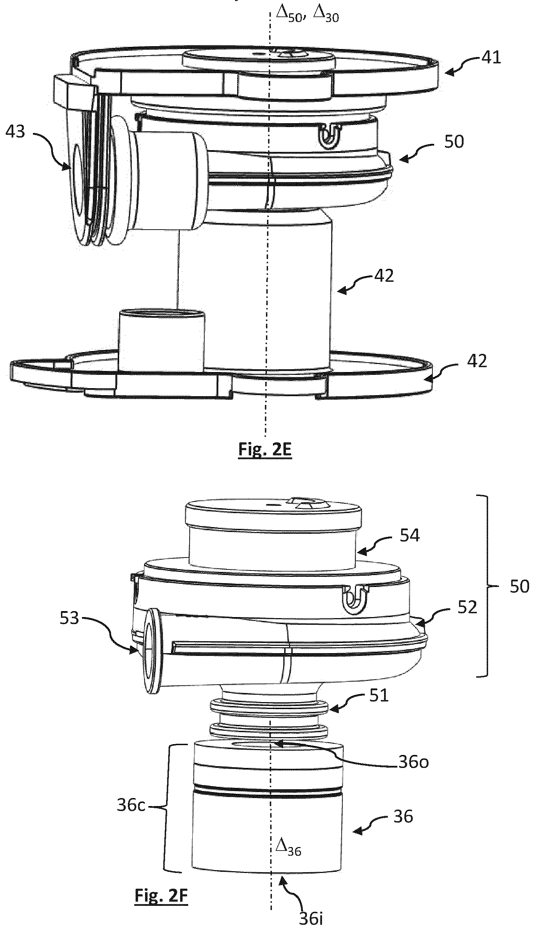

La figure 1E représente l'intérieur du tronçon intermédiaire 12 et du tronçon supérieur 11. L'air admis dans l'enceinte 10, à travers l'ouverture 10i, s'écoule à travers un tube d'entrée 23, en direction du sommet du dispositif, parallèlement ou sensiblement parallèlement à l'axe longitudinal Z. Par sensiblement parallèlement, on entend parallèlement en admettant une tolérance angulaire de ± 20° ou de ± 10°. Le tube d'entrée 23 s'étend le long d'une coque 30, décrite en lien avec les figures 2A à 2F. Le tube d'entrée 23 débouche dans un espace délimité par la coque 30 et un capot supérieur 21. La coque 30 et le capot supérieur 21 sont réalisés en un matériau de préférence rigide, par exemple un plastique. Sur la figure 1E, on a également représenté un tube de sortie 24, s'étendant le long de la coque 30, à travers lequel s'écoule l'air en direction de la sortie d'air 10o ménagée dans le tronçon inférieur 13. Le tube de sortie 24 débouche, dans le tronçon inférieur 13, par une ouverture 20o. L'ouverture 20o est ménagée au niveau de l'interface entre le tronçon intermédiaire 12 et le tronçon inférieur 13. L'air, débouchant dans le tronçon inférieur 13 par l'ouverture 20o, s'écoule, dans le tronçon inférieur 13, vers l'ouverture de sortie 10o. Le dispositif comporte également une carte électronique auxiliaire 18', reliée au commutateur de mise en service 15. La carte électronique auxiliaire 18' est également reliée à l'unité de contrôle 18. Le dispositif 1 comporte un débitmètre 26, relié à l'unité de contrôle 18. Le débitmètre 26 permet une mesure du débit d'air dans le tube d'entrée 23. Dans cet exemple, le débitmètre 26 est ménagé au niveau du tube d'entrée 23. Le débitmètre 26 permet d'ajuster une pression de consigne de l'air adressé par le dispositif 1 vers le masque 3 de l'utilisateur. Par exemple, en cas d'apnée, le débit d'air chute, ce qui entraîne une augmentation de la pression de consigne.

Le dispositif comporte un capteur de pression 25, mesurant la pression de l'air dans le tronçon inférieur 13, en amont de l'ouverture de sortie 10o. En fonction de la pression mesurée, l'unité de contrôle 18 adapte la puissance du ventilateur de façon à maintenir une pression aussi stable que possible autour de la pression de consigne.

Un élément important de l'invention est la présence d'une coque 30, disposée à l'intérieur de l'enceinte 11. Ainsi, l'enceinte 11 enveloppe totalement la coque 30. La coque 30 s'étend autour d'un axe central Δ₃₀. De préférence, l'axe central Δ₃₀ est parallèle, voire confondu, avec l'axe longitudinal Z selon lequel s'étend l'enceinte 10. Les figures 2A et 2B sont des vues de l'extérieur de la coque 30. Dans l'exemple représenté, la coque 30 s'étend, selon l'axe central Δ₃₀, entre une première ouverture 30i, permettant l'admission d'air à l'intérieur de la coque, et un fond de coque 33, plein. La coque comporte une partie supérieure 31, une partie intermédiaire 32 ainsi que le fond de coque 33, assemblés deux à deux, selon l'axe central Δ₃₀.

Entre la partie supérieure 31 et la partie intermédiaire 32 s'étend une première membrane 41, formant un joint entre la partie supérieure 31 et la partie intermédiaire 32. La première membrane est une membrane souple, réalisée par exemple en un matériau élastomère, par exemple à base de silicone. Entre la partie intermédiaire 32 et le fond de coque 33 s'étend une deuxième membrane 42, formant un joint entre la partie intermédiaire 32 et le fond de coque 33. La deuxième membrane 42 est une membrane souple, réalisée par exemple en un matériau élastomère, par exemple à base de silicone.

Le tube d'entrée 23 et le tube de sortie 24 forment une pièce unique avec la partie intermédiaire 32. Cela permet de réduire le nombre de pièces formant le dispositif.

L'air débouchant du tube d'entrée 23 s'écoule dans un espace délimité par la partie supérieure 31 et le capot 21. Il pénètre dans la coque 30 par l'ouverture d'admission 30i, comme le montrent les flèches grises de la figure 2A et 2B. L'air s'extrait de la coque 30 par une deuxième ouverture, formant une ouverture d'extraction 30o, ménagée dans la partie intermédiaire 32 de la coque 30. L'ouverture d'extraction 30o est indiquée sur la figure 2B. Elle s'étend parallèlement à l'axe central Δ₃₀.

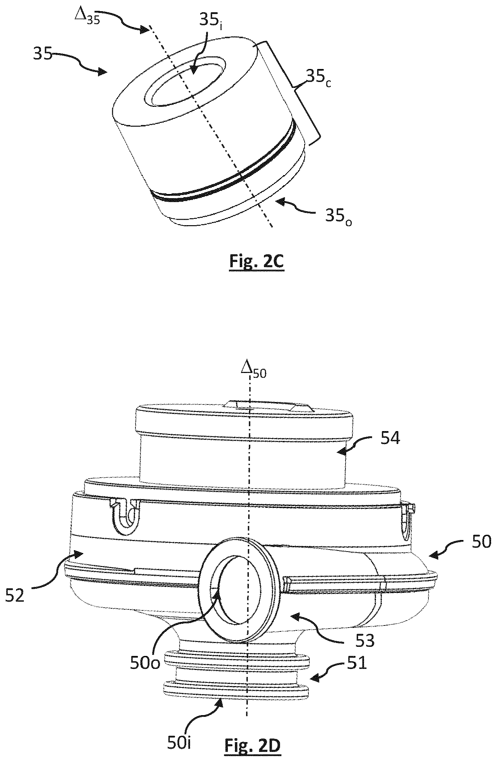

De façon avantageuse, une chambre d'expansion, dite chambre d'expansion amont 35 s'étend au niveau de l'ouverture d'admission 30i. Le terme chambre d'expansion désigne une chambre s'étendant entre une entrée de chambre et une sortie de chambre, le long d'un axe. Comme représenté sur la figure 2C, la chambre d'expansion 35 est telle qu'elle comporte une partie centrale 35c, entre l'entrée de chambre 35i et la sortie de chambre 35o, de telle sorte que perpendiculairement à l'axe de la chambre Δ₃₅, désigné par le terme axe amont, la section de la partie centrale est strictement supérieure à la section de l'entrée et à la section de sortie de chambre. Par section, il est entendu la surface délimitée par la chambre d'expansion, perpendiculairement à l'axe autour duquel s'étend la chambre. Ainsi, la section de la partie centrale est au moins 1.5 fois plus importante, voire au moins deux ou trois fois plus importante que la section de l'entrée et de la sortie de la chambre d'expansion. La section centrale peut être partiellement remplie d'une mousse acoustique, par exemple une mousse à pores ouverts. Le recours à une telle chambre d'expansion permet de réduire significativement le bruit d'écoulement d'air.

De façon plus générale, le recours à une circulation d'air à travers des sections comportant des élargissements suivis de rétrécissements permet une réduction du bruit d'écoulement d'air.

Les figures 2D et 2E représentent les principaux éléments disposés à l'intérieur de la coque 30. La coque 30 renferme une chambre de ventilation 50, dans laquelle s'étend un ventilateur. Le ventilateur est l'accessoire essentiel du dispositif, car il assure la circulation d'air, entre l'entrée 10i de l'enceinte et la sortie 10o. Plus précisément, le ventilateur permet l'aspiration de l'air, à travers de l'entrée 10i du dispositif, et l'insufflation de l'air à partir de la sortie 10o du dispositif. La chambre de ventilation 50 comporte, dans cet exemple, quatre parties :
- une portion d'admission 51, de forme tubulaire, et permettant l'admission de l'air dans la chambre de ventilation à travers une entrée 50i;
- un carter arrondi 52 enveloppant le ventilateur, les pales de ce dernier tournant à l'intérieur du carter arrondi ;
- une portion d'évacuation 53, de forme tubulaire, débouchant sur une sortie 50o de la chambre de ventilation 50 ;
- un compartiment moteur 54, relié électriquement au circuit de contrôle 18.

Le carter 52 et la portion d'évacuation 53 s'étendent perpendiculairement à la portion d'admission 51. Le carter 52, la portion d'admission 51, et le compartiment moteur 54 s'étendent autour d'un axe Δ₅₀, qui correspond à l'axe de rotation du ventilateur.

De préférence, l'axe de rotation Δ₅₀ du ventilateur est confondu avec l'axe longitudinal Z de l'enceinte 10 et/ou avec l'axe central Δ₃₀ de la coque 30. Ainsi, l'axe de rotation Δ₅₀ du ventilateur correspond à l'axe longitudinal Z de l'enceinte. Par conséquent, lorsque l'axe longitudinal Z de l'enceinte 10 est vertical, ou sensiblement vertical, ce qui correspond à la position de fonctionnement du dispositif 1. Les pales du ventilateur s'étendent dans un plan horizontal et tournent selon ce dernier, à l'intérieur du carter 52.

Un aspect remarquable du dispositif est qu'il comporte ainsi trois parties emboîtées les unes dans les autres :
- l'enceinte 10, qui s'étend autour de l'axe longitudinal Z ;
- la coque 30, incluse dans l'enceinte 10, et qui s'étend autour de l'axe central Δ₃₀, et qui est incluse dans l'enceinte 10
- la chambre de ventilation 50, comportant le ventilateur, qui s'étend autour de l'axe de rotation Δ₅₀ du ventilateur, et qui est incluse dans la coque.

De tels emboîtements, en "poupée russe", permet de former une série de chicanes, pour contraindre l'air à effectuer une série de zigzags entre l'entrée 10i de l'enceinte 10 et le ventilateur. Une telle circulation d'air, à l'intérieur de l'enceinte 10, permet d'obtenir un dispositif particulièrement silencieux. On remarque d'ailleurs que les entrées respectives de l'enceinte 10, de la coque 30 et de la chambre de ventilation 50 ne sont pas alignées. Ainsi, l'entrée 10i de l'enceinte, qui forme l'entrée du dispositif 1, est disposée sur une face latérale de l'enceinte, parallèle ou sensiblement parallèle à l'axe longitudinal Z. L'ouverture d'admission 30i de la coque 30 est ménagée autour de l'axe longitudinal Z, dans un plan perpendiculaire ou sensiblement perpendiculaire à l'axe central Δ₃₀, ce dernier étant de préférence confondu à l'axe longitudinal Z. Face à l'entrée 30i de la coque 30 se trouve le capot supérieur 21, ce dernier étant plein. Le capot supérieur 21 forme un déflecteur d'air. Le flux d'air débouchant du tube d'entrée 23 est donc contraint à une première rotation de 180° pour s'engager dans la coque 30. Le flux d'air est alors aspiré vers la chambre de ventilation 50, dont l'entrée 50i est orientée autour de l'axe de rotation Δ₅₀, ce dernier étant de préférence aligné à l'axe central Δ₃₀ et à l'axe longitudinal Z. L'entrée 50i de la chambre de ventilation 50 est disposée en face du fond de coque 33. Ce dernier étant plein, il forme un déflecteur d'air. Ainsi, le flux d'air est contraint à une deuxième rotation à 180° de façon à s'engager à travers l'entrée 50i de la chambre de ventilation.

L'emboîtement de la coque 30 à l'intérieur de l'enceinte 10 permet également d'atténuer le bruit généré par le dispositif, l'enceinte 10 atténuant le bruit généré à l'intérieur de la coque 30.

Afin de limiter le bruit et d'atténuer au maximum les vibrations mécaniques induites par le fonctionnement du ventilateur, la chambre de ventilation 50 est maintenue en suspension par la première membrane 41 et la deuxième membrane 42. La première membrane 41 et la deuxième membrane 42 sont formées d'un matériau souple, typiquement un élastomère, par exemple à base de silicone. La fonction de ces membranes est triple :
- assurer un maintien de la chambre de ventilation en atténuant les vibrations du ventilateur, parallèlement à l'axe de rotation ou perpendiculairement à ce dernier : cela améliore le silence de fonctionnement du dispositif et permet d'éviter un contact entre la chambre de ventilation et la coque ;
- définir la circulation de l'air à l'intérieur de la chambre 30, en amont de la chambre de ventilation, les membranes présentant des ouvertures 41₁ 42₁ formant des passages à travers lesquels le flux d'air s'écoule ;
- former des joints entre les différentes parties constituant la coque 30, respectivement entre la partie supérieure 31 et la partie intermédiaire 32, et entre la partie intermédiaire 32 et le fond de coque 33.

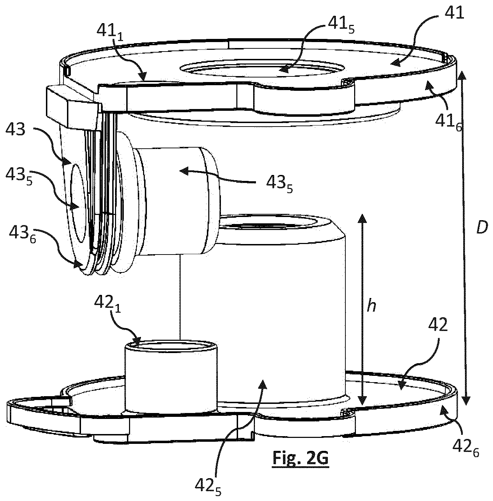

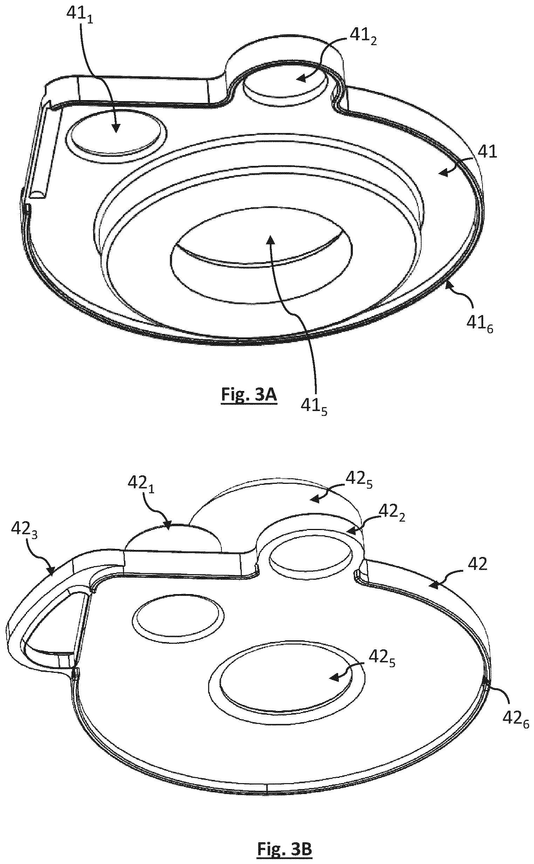

La figure 2E montre la disposition de la première membrane 41 et de la deuxième membrane 42 à l'intérieur de la coque 30. Comme représenté sur les figures 2E, 2G et 3A, la première membrane 41 s'étend entre une périphérie 41₆, dite première périphérie, fixée à la coque 30, et la chambre de ventilation 50. La chambre de ventilation 50 est insérée dans une première ouverture centrale 41₅. La première périphérie 41₆ est maintenue entre la partie supérieure 31 et la partie intermédiaire 32 de la coque 30. La première ouverture centrale 41s permet l'insertion et le maintien de la chambre de ventilation 50. Lors de l'insertion de la chambre de ventilation 50, la première ouverture centrale 41s est déformée élastiquement et s'ajuste autour de la chambre de ventilation 50, et plus précisément autour du carter 52 ou du compartiment moteur 54. Dans ce mode de réalisation, la première ouverture centrale 41s est directement en contact avec la chambre de ventilation 50, bien qu'il soit possible de disposer un support entre la première ouverture centrale 41₅ et la chambre de ventilation 50. Le maintien de la chambre de ventilation 50 par la première membrane 41 est assuré par la tension de la première membrane 41, entre la première périphérie 41₆ et la première ouverture centrale 41₅. Ainsi, la première membrane 41 fait office de support de la chambre de ventilation. Entre la chambre de ventilation 50 et la première périphérie 41₆, la première membrane 41 s'étend perpendiculairement à l'axe de rotation Δ₅₀, ainsi qu'à l'axe central Δ₃₀ et à l'axe longitudinal Z.

Comme représenté sur les figures 2E, 2G et 3B, la deuxième membrane 42 s'étend entre une périphérie 42₆, dite deuxième périphérie, raccordée à la coque 30, et la chambre de ventilation 50, et plus précisément la portion d'admission 51 de la chambre d'admission 50. La deuxième périphérie 42₆ est maintenue entre la partie intermédiaire 32 et le fond de coque 33. La deuxième membrane 42 comporte une ouverture centrale 42s, dite deuxième ouverture centrale. La deuxième ouverture centrale 42₅ permet l'insertion et le maintien du support de la chambre de ventilation 50. Le maintien de la chambre de ventilation est assuré :
- soit directement par la deuxième membrane 42, cette dernière s'étendant au contact de la chambre de ventilation 50, et notamment au niveau de la portion d'admission 51, ce qui correspond à l'exemple représenté ;
- soit par un support, interposé entre la deuxième membrane 42 et la portion d'admission 51 de la chambre de ventilation 50.

Dans cet exemple, le dispositif 1 comporte une chambre d'expansion 36, insérée à travers la deuxième ouverture centrale 42₅ de la deuxième membrane 42. La chambre d'expansion 36, désignée par le terme chambre d'expansion aval, est similaire à la chambre d'expansion amont 35 préalablement décrite. Elle comporte une partie centrale 36c, entre une entrée de chambre 36i et la sortie de chambre 36o, de telle sorte que perpendiculairement à un axe aval Δ₃₆, la section de la partie centrale 36c est strictement supérieure à la section de l'entrée et à la section de sortie de chambre. La sortie 36o de la chambre d'expansion aval s'engage dans ou autour de la portion d'admission 51 de la chambre de ventilation 50. De même que la chambre d'expansion amont 35, la chambre d'expansion aval 36 contribue à réduire le bruit d'écoulement d'air. Dans l'exemple représenté, l'axe amont Δ₃₅ et l'axe aval Δ₃₆ sont parallèles à l'axe central Δ₃₀ de la coque 30.

L'exemple présenté dans cette description comporte deux chambres d'expansion 35 et 36, disposées en série en amont de la chambre de ventilation. Selon d'autres modes de réalisation, une seule chambre d'expansion serait utilisable. Cependant, il est estimé que la présence de deux chambres d'expansion permet une réduction plus efficace du bruit dû à l'écoulement d'air.

La deuxième ouverture centrale 42₅ de la deuxième membrane 42 est tubulaire. Elle s'étend, selon l'axe de rotation Δ₅₀ (ou l'axe central Δ₃₀), selon une hauteur h typiquement comprise entre 5 mm et 6 cm, par exemple 3.5 cm. Cela permet une meilleure préhension de la chambre d'expansion 36. La hauteur h est représentée sur la figure 2G.

Au niveau de leurs périphéries respectives 41₆ et 42₆, la première membrane 41 et la deuxième membrane 42 sont distantes, selon l'axe central Δ₃₀, d'une distance D de préférence comprise entre 1 cm et 5 cm. La distance D est représentée sur la figure 2G. Les périphéries 41₆ et 42₆ forment des points d'accroche de la chambre de ventilation 50 sur la coque 30. La coque 30 étant fixe par rapport à l'enceinte 10, les première et deuxième périphéries sont fixes par rapport à l'enceinte 10. Le fait de maintenir une distance D entre ces points d'accroche favorise un bon maintien de la chambre de ventilation, et une bonne atténuation des vibrations de la chambre de ventilation induites durant le fonctionnement du ventilateur. Les inventeurs ont d'ailleurs constaté que le maintien de la chambre de ventilation par deux membranes souples distantes l'une de l'autre, au moins au niveau de leurs périphéries respectives, permet d'éviter le recours à des membranes comportant un ou plusieurs soufflets. On sait que de tels soufflets permettent une atténuation efficace des vibrations, mais ils constituent une certaine zone de fragilité et augmentent le coût de fabrication.

Le maintien de la chambre de ventilation 50 par deux membranes distantes l'une de l'autre, au niveau de leurs périphéries respectives, permet une limitation du déplacement de la chambre de ventilation parallèlement à l'axe central Δ₃₀ de la coque 30. On évite par exemple tout choc entre la chambre de ventilation 50 et une partie rigide du dispositif. Cela permet également d'éviter un choc du ventilateur avec les parois rigides de la coque 30 lors du transport du dispositif. Cette conception permet donc d'obtenir un dispositif robuste. Cela évite la présence de moyens d'amortissement disposés sur la chambre de ventilation, comme décrit en lien avec l'art antérieur.

Le dispositif comporte une troisième membrane 43, optionnelle, s'étendant essentiellement parallèlement à l'axe central Δ₃₀ de la coque 30. Elle comporte une ouverture centrale 43₅, dite troisième ouverture centrale, s'engageant autour de la portion d'évacuation 53 de la chambre de ventilation 50. La troisième membrane 43 forme ainsi un joint, entre la portion d'évacuation 53 de la chambre de ventilation 50 et le tube de sortie 24. L'air passe ainsi de la portion d'évacuation 53, à travers l'ouverture d'extraction 30o de la coque 30, vers le tube de sortie 24. Dans l'exemple représenté sur la figure 2G, la troisième membrane 43 présente une portion tubulaire 43₅ s'engageant autour de la portion d'évacuation 53. Cela permet un maintien de la chambre de ventilation 50, en atténuant les vibrations de cette dernière sous l'effet du fonctionnement du ventilateur. La troisième membrane 43 présente une périphérie 43₆, dite troisième périphérie 43₆, solidaire de la coque 30. Plus précisément, la troisième périphérie est insérée à travers l'ouverture d'extraction 30o de la coque 30. La troisième membrane 43 peut être solidaire de la première membrane 41, ou distincte de cette dernière.

Les figures 3A et 3B montrent respectivement la première membrane 41 et la deuxième membrane 42. Outre les périphéries 41₆, 42₆ et les ouvertures centrales 41₅, 42₅ précédemment décrites, les membranes comportent d'autres ouvertures agissant sur la circulation d'air à l'intérieur de la coque. La première membrane 41 comporte une ouverture 41₁, s'étendant dans la coque 30, et formant un passage d'air. Dans cette partie de la coque 30, la première membrane 41 forme une cloison interne s'étendant perpendiculairement à l'axe central Δ₃₀ de la coque 30. L'ouverture 41₁ forme une restriction d'air, l'air s'écoulant de part et d'autre de la première membrane 41, ce qui contribue à une atténuation du bruit d'écoulement d'air. De même, la deuxième membrane 42 comporte une ouverture 42₁, s'étendant dans la coque 30, et formant un passage d'air. Dans cette partie de la coque 30, la deuxième membrane 42 forme une cloison interne s'étendant perpendiculairement à l'axe central Δ₃₀. Cette ouverture forme une restriction d'air, l'air s'écoulant de part et d'autre de la deuxième membrane 42, ce qui contribue à une atténuation du bruit d'écoulement d'air.

Outre les ouvertures décrites ci-dessous, la première et la deuxième membranes définissent d'autres ouvertures structurelles, permettant de se conformer à la structure du dispositif 1. Ainsi:
- la première membrane 41 comporte une ouverture structurelle 41₂, s'étendant autour du tube d'entrée 23 au niveau de l'interface entre le tube d'entrée 23 et le capot supérieur 21. La première membrane 41 fait ici office de joint entre le tube d'entrée 23 et le capot supérieur 21 ;
- la deuxième membrane comporte une première ouverture structurelle 42₂, s'étendant autour du tube d'entrée 23, au niveau de l'interface entre la partie intermédiaire 32 de la coque 30 et le fond de coque 33.
- la deuxième membrane comporte une deuxième ouverture structurelle 42₃, s'étendant autour du tube de sortie 24.

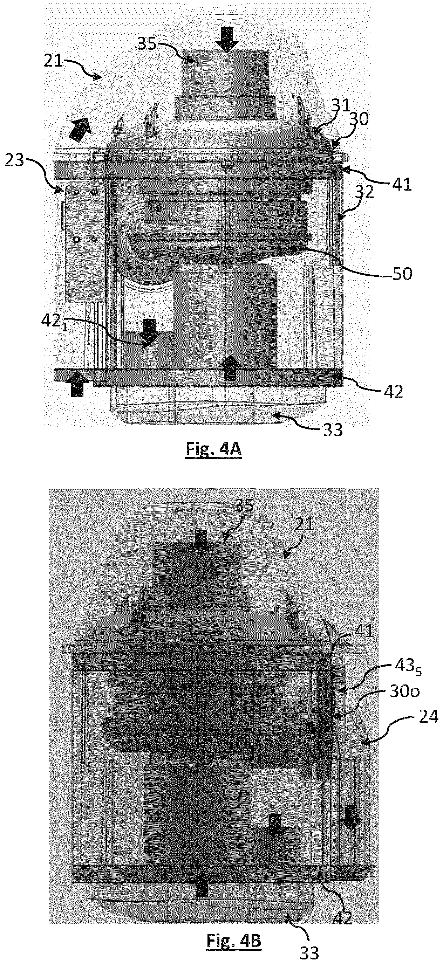

Les figures 4A et 4B permettent une représentation de la circulation d'air à l'intérieur de l'enceinte, la circulation étant représentée par des flèches noires : après avoir été admis dans l'enceinte 10, l'air s'écoule à travers le tube d'entrée 23, puis pénètre dans la chambre d'expansion amont 35 après avoir été défléchi par le capot supérieur 21. Il pénètre ensuite à l'intérieur de la coque 30 et s'écoule ensuite à travers les ouvertures 41₁ et 42₁ respectivement ménagées dans les première et deuxième membranes 41 et 42. Il est ensuite défléchi par le fond de coque 33, en direction de la chambre d'expansion aval 36. Il pénètre ensuite à l'intérieur de la chambre de ventilation 50. Entre l'admission à l'intérieur de l'enceinte et la chambre de ventilation 50, l'air est aspiré par la dépression générée par le ventilateur. En amont du ventilateur, le dispositif définit ainsi une zone amont de circulation d'air, s'étendant entre l'entrée 10i et la chambre de ventilation 50, respectivement à travers le tube d'entrée 23 et les différentes parties de la coque 30. L'air est ensuite soufflé par le ventilateur. Il ressort de la chambre de ventilation à travers l'ouverture 43₅ de la troisième membrane 43, et s'écoule, à travers le tube de sortie 24, jusqu'au tronçon inférieur 13 de l'enceinte 10. En aval du ventilateur, le dispositif définit ainsi une zone avale de circulation d'air, s'étendant entre la chambre de ventilation 50 et la sortie 10o, en particulier à travers le tube de sortie 24.

Dans le tronçon inférieur 13 de l'enceinte 10, comme précédemment évoqué, l'air peut être humidifié dans un humidificateur, avant de sortir de l'enceinte 10 par l'ouverture de sortie 10o. Il est alors dirigé vers le masque respiratoire 3, à travers le tuyau souple 2.

On remarque la forme arrondie des principaux composants du dispositif 1 représentés sur les figures 4A et 4B. Il s'agit notamment du capot supérieur 21 et de la coque 30. Une telle forme est propice à une variation de la section à travers laquelle s'écoule l'air. Cela permet notamment de ménager des élargissements et rétrécissements successifs, tout le long de la circulation d'air à travers l'enceinte 10. Comme précédemment indiqué, cela diminue le bruit de l'écoulement d'air. Ce bruit peut être davantage atténué en disposant de la mousse le long des parois du capot supérieur 21, ou de la paroi interne de la coque 30. La forme arrondie augmente également la rigidité.

On observe que le dispositif comporte un nombre réduit de composants essentiels, et que leur forme est simple. Cela permet une réalisation par des techniques d'impression 3D ou par moulage, à un coût maîtrisé. Dans l'exemple représenté, le tube d'arrivée 23 et le tube de sortie 24 sont réalisés sur la même pièce que le tronçon intermédiaire 32 de la coque. Par ailleurs, les première et deuxième membranes assurent de multiples fonctions, telles que précédemment décrites. La forme de ces membranes est relativement simple, ce qui facilite leur fabrication. Le dispositif est ainsi réalisable à moindre coût de fabrication.

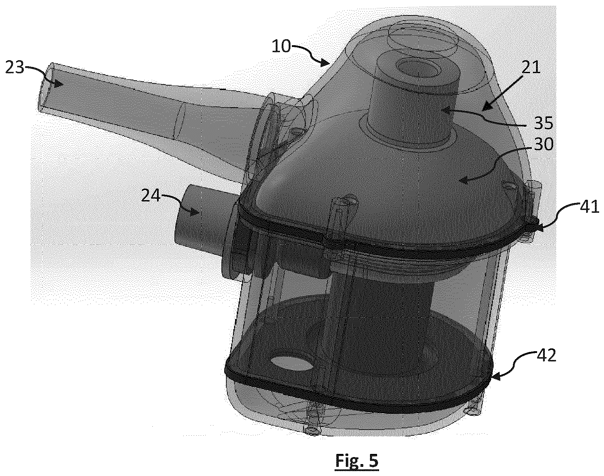

La figure 5 schématise un deuxième mode de réalisation, similaire au mode de réalisation décrit en lien avec les figures 1 à 4. Une différence notable est l'orientation du tube d'arrivée 23 et du tube de sortie 24. Ces derniers s'étendant perpendiculairement à l'axe longitudinal Z.

## Revendications

1. Dispositif de ventilation respiratoire (1), destiné à envoyer un flux d'air, généré par un ventilateur, dans un conduit (2), le conduit s'étendant entre le dispositif et un masque respiratoire (3), destiné à être utilisé par un utilisateur, le dispositif comportant une enceinte (10), s'étendant autour d'un axe longitudinal (Z) et comprenant :
- une entrée d'air (10i), destinée à admettre l'air dans l'enceinte (10);
- une zone amont de circulation d'air s'étendant entre l'entrée d'air et le ventilateur ;
- une sortie d'air (10o), la sortie d'air étant configurée pour être raccordée au conduit (2);
de telle sorte que lorsque le ventilateur fonctionne, l'air s'écoule depuis l'entrée d'air (10i) successivement à travers la zone amont de circulation d'air, le ventilateur et la sortie d'air, le dispositif étant tel que :
- le ventilateur est disposé dans une chambre de ventilation (50);
- la chambre de ventilation est maintenue par une première membrane (41), souple, s'étendant entre une première périphérie (41₆) et la chambre de ventilation (50), la première périphérie étant maintenue fixe par rapport à l'enceinte (10), la première membrane (41) définissant une première ouverture centrale (41₅), à travers laquelle s'étend la chambre de ventilation (50), ou un support relié à la chambre de ventilation ;
le dispositif étant **caractérisé en ce que** :
- la chambre de ventilation (50) est maintenue par une deuxième membrane souple (42), distincte de la première membrane (41), la deuxième membrane s'étendant entre une deuxième périphérie (42₆) et la chambre de ventilation (50), la deuxième périphérie (42₆) étant maintenue fixe par rapport à l'enceinte (10), à une distance non nulle de la première périphérie (41₆), la deuxième membrane comportant une deuxième ouverture centrale (42₅), à travers laquelle s'étend la chambre de ventilation ou un support relié à la chambre de ventilation (50).

2. Dispositif selon la revendication 1, dans lequel :
- la chambre de ventilation (50) comporte une portion d'admission (51), un carter du ventilateur (52), une portion d'évacuation (53) et un compartiment moteur (54) ;
- la première membrane (41) s'étend entre la première périphérie (41₆) et le carter du ventilateur (52) ou entre la première périphérie et le compartiment moteur (54).
- la deuxième membrane (42) s'étend entre la deuxième périphérie (42₆) et la portion d'admission (51).

3. Dispositif selon la revendication 2, comportant une troisième membrane (43), s'étendant entre une troisième périphérie (43₆), fixe par rapport à l'enceinte (10), et la portion d'évacuation (53).

4. Dispositif selon l'une quelconque des revendications 1 à 3, comportant une coque (30), s'étendant autour d'un axe central (Δ₃₀), la coque étant disposée dans l'enceinte (10) en étant fixe par rapport à cette dernière, la coque s'étendant autour de la chambre de ventilation (50), la coque comportant une ouverture d'admission (30i), permettant une admission de l'air dans la coque, et une ouverture d'extraction (30o), permettant l'extraction de l'air de la coque, de telle sorte que la zone amont de circulation d'air s'étend:
- à l'extérieur de la coque, entre l'entrée d'air de l'enceinte (10i) et l'ouverture d'admission (30i);
- à l'intérieur de la coque, entre l'ouverture d'admission (30i) et la chambre de ventilation (50).

5. Dispositif selon la revendication 4, dans lequel :
- la coque (30) comporte un fond de coque (33), en face de l'ouverture d'admission (30i);
- le fond de coque (33) fait face à la portion d'admission (51) de la chambre de ventilation (50), le fond de coque formant un déflecteur d'air entre l'ouverture d'admission (30i) et la portion d'admission (51).

6. Dispositif selon la revendication 5, dans lequel l'axe central de la coque (Δ₃₀) correspond à l'axe longitudinal (Z), l'ouverture d'admission (30i) et le fond de coque (33) étant alignés selon l'axe longitudinal (Z).

7. Dispositif selon l'une quelconque des revendications 4 à 6, dans lequel la première membrane (41) est maintenue par la coque (30), et s'étend entre la coque et la chambre de ventilation (50), la première membrane s'étendant, au moins en partie, perpendiculairement à l'axe central de la coque (Δ₃₀).

8. Dispositif selon la revendication 7, dans lequel la première membrane (41) forme une première cloison à l'intérieur de la coque, la première membrane comportant une ouverture (41₁) de façon à permettre un passage de l'air de part et d'autre de la première cloison.

9. Dispositif selon l'une quelconque des revendications 4 à 8, dans lequel la deuxième membrane (42) est maintenue par la coque (30), et s'étend entre la coque et la chambre de ventilation (50), la deuxième membrane s'étendant, au moins en partie, perpendiculairement à l'axe central de la coque (Δ₃₀).

10. Dispositif selon la revendication 9, dans lequel la deuxième membrane forme une deuxième cloison à l'intérieur de la coque, la deuxième membrane comportant une ouverture (42₁) de façon à permettre un déplacement de l'air de part et d'autre de la deuxième cloison.

11. Dispositif selon l'une quelconque des revendications 4 à 10, dans lequel la portion d'évacuation (52) de la chambre de ventilation :
- débouche dans l'ouverture d'extraction (30o) de la coque ;
- ou s'étend à travers l'ouverture d'extraction (30o) de la coque.

12. Dispositif selon l'une quelconque des revendications 4 à 11, comportant une chambre d'expansion amont (35), adjacente de l'ouverture d'admission (30i) de la coque, la chambre d'expansion amont comportant une entrée (35i), une partie centrale (35c), et une sortie (35o), agencées autour d'un axe amont (Δ₃₅), la section de la partie centrale, perpendiculairement à l'axe amont, étant supérieure aux sections respectives de l'entrée et de la sortie de la chambre d'expansion d'amont.

13. Dispositif selon la revendication 12, dans laquelle la chambre d'expansion amont débouche sur l'ouverture d'admission de la coque.

14. Dispositif selon l'une quelconque des revendications 2 à 13, comportant une chambre d'expansion aval (36), adjacente de la portion d'admission (51) de la chambre de ventilation, la chambre d'expansion aval comportant une entrée (36i), une partie centrale (36c), et une sortie (36o), agencées autour d'un axe aval (Δ₃₆), la section de la partie centrale, perpendiculairement à l'axe aval, étant supérieure aux sections respectives de l'entrée et de la sortie de la chambre d'expansion aval.

15. Dispositif selon la revendication 14, dans lequel :
- la chambre d'expansion aval (36) débouche sur la portion d'admission (51) de la chambre de ventilation;
- la deuxième membrane (42) s'étend entre la deuxième périphérie (42₆) et la chambre d'expansion aval (36), de telle sorte que la portion d'admission est maintenue par la deuxième membrane.

16. Dispositif selon l'une quelconque des revendications précédentes, dans lequel la première membrane (41) et la deuxième membrane (42) sont souples et formées d'un matériau élastomère.

## Patentansprüche

1. Lungenventilationsvorrichtung (1), die für das Schicken eines durch einen Ventilator erzeugten Luftstroms durch eine Leitung (2) bestimmt ist, wobei die Leitung sich zwischen der Vorrichtung und einer Atemmaske (3), die für das Tragen durch einen Benutzer bestimmt ist, erstreckt,, wobei die Vorrichtung ein Gehäuse (10) umfasst, das sich um eine Längsachse (Z) erstreckt, wobei die Vorrichtung umfasst:
- einen Lufteinlass (10i), der dazu bestimmt ist, Luft in das Gehäuse (10) einzulassen;
- eine vorgeschaltete Luftzirkulationszone, die sich zwischen dem Lufteinlass und dem Ventilator erstreckt;
- einen Luftauslass (10o), wobei der Luftauslass zum Anschließen an die Leitung (2) konfiguriert ist :
so dass, wenn der Ventilator läuft, die Luft vom Lufteinlass weiter zum Ventilator und dann zur vorgeschalteten Luftzirkulationszone und zum Luftauslass strömt, wobei die Vorrichtung so beschaffen ist, dass:
- der Ventilator ist in einer Ventilationskammer (50) angeordnet;
- die Ventilationskammer durch eine erste flexible Membran (41) gehalten wird, die sich zwischen einer ersten Peripherie (41₆ ) und der Ventilationskammer (50) erstreckt, wobei die erste Peripherie in Bezug auf das Gehäuse (10) fest gehalten wird, wobei die erste Membran (41) eine erste zentrale Öffnung (41₅ ) aufweist, durch die sich die Ventilationskammer (50) oder ein mit der Ventilationskammer verbundener Träger erstreckt;
wobei die Vorrichtung **dadurch gekennzeichnet ist, dass**:
- die Ventilationskammer (50) durch eine zweite flexible Membran (42) gehalten wird, die von der ersten Membran (41) getrennt ist, wobei sich die zweite Membran zwischen einer zweiten Peripherie (42₆ ) und der Ventilationskammer (50) erstreckt, wobei die zweite Peripherie (42₆ ) relativ zu dem Gehäuse (10) in einem von Null verschiedenen Abstand von der ersten Peripherie (41₆ ) fest gehalten wird, wobei die zweite Membran eine zweite zentrale Öffnung (42₆ ) aufweist, durch die sich die Ventilationskammer oder ein mit der Ventilationskammer (50) verbundener Träger erstreckt.

2. Die Vorrichtung nach Anspruch 1, wobei:
- die Ventilationskammer (50) einen Ansaugbereich (51), ein Ventilatorgehäuse (52), einen Auslassbereich (53) und einen Motorraum (54) umfasst;
- die erste Membran (41) sich zwischen der ersten Peripherie (41₆ ) und dem Ventilatorgehäuse (52) oder zwischen der ersten Peripherie und dem Motorraum (54) erstreckt;
- die zweite Membran (42) sich zwischen der zweiten Peripherie (42₆ ) und dem Ansaugbereich (51) erstreckt.

3. Vorrichtung nach Anspruch 2, mit einer dritten Membran (43), die sich zwischen einem dritten Peripherie (43₆ ), der relativ zum Gehäuse (10) befestigt ist, und dem Auslassbereich (53) erstreckt.

4. Vorrichtung nach einem der Ansprüche 1 bis 3, umfassend eine Schale (30), die sich um eine zentrale Achse (Δ₃₀ ) erstreckt, wobei die Schale in dem Gehäuse (10) angeordnet ist, indem sie relativ dazu befestigt ist, wobei sich die Schale um die Ventilationskammer (50) herum erstreckt, wobei die Schale eine Einlassöffnung (30i) umfasst, um Luft in die Schale einzulassen, und eine Auslassöffnung (30o), um Luft aus der Schale auszulassen, so dass sich die vorgeschaltete Luftzirkulationszone erstreckt:
- außerhalb der Schale, zwischen dem Lufteinlass des Gehäuses (10i) und der Einlassöffnung (30i);
- im Inneren der Schale, zwischen der Einlassöffnung (30i) und der Ventilationskammer (50).

5. Vorrichtung nach Anspruch 4, wobei:
- die Schale (30) einen Schalenboden (33) gegenüber der Einlassöffnung (30i) aufweist;
- der Schalenboden (33) dem Ansaugbereich (51) der Ventilationskammer (50) zugewandt ist, wobei der Schalenboden einen Luftabweiser zwischen der Einlassöffnung (30i) und dem Ansaugbereich (51) bildet.

6. Vorrichtung nach Anspruch 5, wobei die zentrale Achse (Δ₃₀) der Schale der Längsachse (Z) entspricht, wobei die Einlassöffnung (30i) und der Schalenboden (33) entlang der Längsachse (Z) ausgerichtet sind.

7. Vorrichtung nach einem der Ansprüche 4 bis 6, wobei die erste Membran (41) von der Schale (30) gehalten wird und sich zwischen der Schale und der Ventilationskammer (50) erstreckt, wobei die erste Membran zumindest teilweise senkrecht zur zentrale Achse (Δ₃₀ ) der Schale verläuft.

8. Vorrichtung nach Anspruch 7, wobei die erste Membran (41) eine erste Trennwand innerhalb der Schale bildet, wobei die erste Membran eine Öffnung (41₁) aufweist, so dass Luft auf beiden Seiten der ersten Trennwand bewegen kann.

9. Vorrichtung nach einem der Ansprüche 4 bis 8, wobei die zweite Membran (42) von der Schale (30) gehalten wird und sich zwischen der Schale und der Ventilationskammer (50) erstreckt, wobei die zweite Membran zumindest teilweise senkrecht zur zentrale Achse (Δ₃₀ ) der Schale verläuft.

10. Vorrichtung nach Anspruch 9, wobei die zweite Membran eine zweite Trennwand innerhalb der Schale bildet, wobei die zweite Membran eine Öffnung (42₁) aufweist, so dass Luft auf beiden Seiten der zweiten Trennwand bewegen kann.

11. Vorrichtung nach einem der Ansprüche 4 bis 10, wobei der Auslassbereich (52) der Ventilationskammer:
- tritt in der Auslassöffnung (30o) der Schale aus;
- oder sich durch die Auslassöffnung (30o) der Schale erstreckt.

12. Vorrichtung nach einem der Ansprüche 4 bis 11, umfassend eine vorgeschaltete Expansionskammer (35), die an die Einlassöffnung (30i) der Schale angrenzt, wobei die vorgeschaltete Expansionskammer einen Einlass (35i), einen zentralen Teil (35c) und einen Auslass (35o) umfasst, die um eine vorgeschaltete Achse (Δ₃₅ ) angeordnet sind, wobei der Querschnitt des zentralen Teils senkrecht zur vorgeschalteten Achse größer ist als die jeweiligen Querschnitte des Einlasses und des Auslasses der vorgeschalteten Expansionskammer.

13. Vorrichtung nach Anspruch 12, wobei die vorgeschaltete Expansionskammer an der Einlassöffnung der Schale austritt.

14. Vorrichtung nach einem der Ansprüche 2 bis 13, umfassend nachgeschaltete Expansionskammer (36), die an den Ansaugbereich (51) der Ventilationkammer angrenzt, wobei die nachgeschaltete Expansionskammer einen Einlass (36i), einen zentralen Teil (36c) und einen Auslass (36o) umfasst, die um eine nachgeschaltete Achse (Δ₃₆) angeordnet sind, wobei der Querschnitt des zentralen Teils senkrecht zur nachgeschalteten Achse größer ist als die jeweiligen Querschnitte des Einlasses und des Auslasses der nachgeschalteten Expansionskammer.

15. Vorrichtung nach Anspruch 14, wobei:
- die nachgeschaltete Expansionskammer (36) mündet in den Ansaugbereich (51) der Ventilationskammer;
- die zweite Membran (42) sich zwischen der zweiten Peripherie (42₆ ) und der nachgeschalteten Expansionskammer (36) erstreckt, so dass der Ansaugbereich von der zweiten Membran zurückgehalten wird.

16. Vorrichtung nach einem der vorhergehenden Ansprüche, wobei die erste Membran (41) und die zweite Membran (42) flexibel sind und aus einem Elastomermaterial gebildet sind.

## Claims

1. A respiratory ventilation device (1) intended to send an air flow, generated by a fan, into a conduit (2), with the conduit extending between the device and a respiratory mask (3) intended to be used by a user, the device comprising an enclosure (10) extending around a longitudinal axis (Z) and comprising:
- an air inlet (10i) intended to admit air into the enclosure (10);
- an upstream air circulation zone extending between the air inlet and the fan;
- an air outlet (10o), the air outlet being configured to be connected to the conduit (2), so that, when the fan operates, air successively flows from the air inlet (10i) through the upstream air circulation zone, the fan and the air outlet, the device being such that:
- the fan is arranged in a ventilation chamber (50);
- the ventilation chamber is retained by a first flexible membrane (41) extending between a first periphery (41₆) and the ventilation chamber (50), the first periphery being fixedly retained relative to the enclosure (10), the first membrane (41) defining a first central opening (41s), through which the ventilation chamber (50), or a support connected to the ventilation chamber, extends;
- the device being **characterized in that**:
- the ventilation chamber (50) is retained by a second flexible membrane (42), separate from the first membrane (41), the second membrane extending between a second periphery (42₆) and the ventilation chamber (50), the second periphery (42₆) being fixedly retained relative to the enclosure (10) at a non-zero distance from the first periphery (41₆), the second membrane comprising a second central opening (42₅), through which the ventilation chamber, or a support connected to the ventilation chamber (50), extends.

2. The device as claimed in claim 1, wherein:
- the ventilation chamber (50) comprises an intake portion (51), a fan housing (52), a discharge portion (53) and a motor compartment (54);
- the first membrane (41) extends between the first periphery (41₆) and the fan housing (52) or between the first periphery and the motor compartment (54);
- the second membrane (42) extends between the second periphery (42₆) and the intake portion (51).

3. The device as claimed in claim 2, comprising a third membrane (43) extending between a third periphery (43₆), which is fixed relative to the enclosure (10), and the discharge portion (53).

4. The device as claimed in any one of claims 1 to 3, comprising a shell (30) extending around a central axis (Δ₃₀), the shell being arranged in the enclosure (10) by being fixed relative thereto, the shell extending around the ventilation chamber (50), the shell comprising an intake opening (30i) allowing air to be admitted into the shell, and an extraction opening (30o) allowing air to be extracted from the shell, so that the upstream air circulation zone extends:
- outside the shell, between the air inlet of the enclosure (10i) and the intake opening (30i);
- inside the shell, between the intake opening (30i) and the ventilation chamber (50).

5. The device as claimed in claim 4, wherein:
- the shell (30) comprises a shell bottom (33) opposite the intake opening (30i);
- the shell bottom (33) faces the intake portion (51) of the ventilation chamber (50), with the shell bottom forming an air deflector between the intake opening (30i) and the intake portion (51).

6. The device as claimed in claim 5, wherein the central axis (Δ₃₀) of the shell corresponds to the longitudinal axis (Z), with the intake opening (30i) and the shell bottom (33) being aligned along the longitudinal axis (Z).

7. The device as claimed in any one of claims 4 to 6, wherein the first membrane (41) is retained by the shell (30) and extends between the shell and the ventilation chamber (50), with the first membrane at least partly extending perpendicular to the central axis (Δ₃₀) of the shell.

8. The device as claimed in claim 7, wherein the first membrane (41) forms a first partition inside the shell, with the first membrane comprising an opening (41₁) so as to allow air to pass on either side of the first partition.

9. The device as claimed in any one of claims 4 to 8, wherein the second membrane (42) is retained by the shell (30) and extends between the shell and the ventilation chamber (50), with the second membrane at least partly extending perpendicular to the central axis (Δ₃₀) of the shell.

10. The device as claimed in claim 9, wherein the second membrane forms a second partition inside the shell, with the second membrane comprising an opening (42₁) so as to allow air to move on either side of the second partition.

11. The device as claimed in any one of claims 4 to 10, wherein the discharge portion (52) of the ventilation chamber:
- emerges in the extraction opening (30o) of the shell;
- or extends through the extraction opening (30o) of the shell.

12. The device as claimed in any one of claims 4 to 11, comprising an upstream expansion chamber (35), adjacent to the intake opening (30i) of the shell, the upstream expansion chamber comprising an inlet (35i), a central part (35c) and an outlet (35o) arranged around an upstream axis (Δ₃₅), with the section of the central part, perpendicular to the upstream axis, being greater than the respective sections of the inlet and of the outlet of the upstream expansion chamber.

13. The device as claimed in claim 12, wherein the upstream expansion chamber emerges at the intake opening of the shell.

14. The device as claimed in any one of claims 2 to 13, comprising a downstream expansion chamber (36), adjacent to the intake portion (51) of the ventilation chamber, the downstream expansion chamber comprising an inlet (36i), a central part (36c) and an outlet (36o) arranged around a downstream axis (Δ₃₆), with the section of the central part, perpendicular to the downstream axis, being greater than the respective sections of the inlet and of the outlet of the downstream expansion chamber.

15. The device as claimed in claim 14, wherein:
- the downstream expansion chamber (36) emerges at the intake portion (51) of the ventilation chamber;
- the second membrane (42) extends between the second periphery (42₆) and the downstream expansion chamber (36), so that the intake portion is retained by the second membrane.

16. The device as claimed in any one of the preceding claims, wherein the first membrane (41) and the second membrane (42) are flexible and are formed from an elastomer material.
